# EUROPÄISCHE PATENTSCHRIFT

(11) **EP 3 389 511 B1**
(45) Veröffentlichungstag und Bekanntmachung des Hinweises auf die Patenterteilung: **01.04.2020**
(21) Anmeldenummer: 16826014.9
(22) Anmeldetag: 14.12.2016
(51) Int. Cl.: A61B 17/12

(54) **IMPLANTAT**
IMPLANT
IMPLANT

(30) Priorität: 14.12.2015 DE 102015121757
(43) Veröffentlichungstag der Anmeldung: 24.10.2018
(73) Patentinhaber: Phenox GmbH, 44801 Bochum (DE)
(72) Erfinder: HENKES, Hans, 70192 Stuttgart (DE); MONSTADT, Hermann, 44797 Bochum (DE); HANNES, Ralf, 44137 Dortmund (DE)
(74) Vertreter: Schneiders & Behrendt PartmbB Rechtsanwälte - Patentanwälte
(86) Internationale Anmeldenummer: PCT/EP2016/080917
(87) Internationale Veröffentlichungsnummer: WO 2017/102804

(56) Entgegenhaltungen:
- WO-A1-2014/029835
- DE-A1-102008 028 308
- US-A1- 2006 064 151
- US-A1- 2007 270 902
- US-A1- 2009 012 559
- US-A1- 2011 046 719

## Beschreibung

Die Erfindung betrifft ein Implantat zum Einsatz bei der Okkludierung von Aneurysmen in Blutgefäßen im Bereich von Gefäßverzweigungen, insbesondere Bifurkationsaneurysmen, wobei das Implantat in einem expandierten Zustand, in dem es im Blutgefäß implantiert wird, und in einem kontrahierten Zustand vorliegt, in dem es durch das Blutgefäß bewegbar ist, wobei das Implantat einen proximalen Fixierabschnitt, mit dem das Implantat an einer Gefäßwand des Blutgefäßes fixierbar ist, einen distalen Abschnitt, in dem das Implantat gegenüber dem Fixierabschnitt radial erweitert ist und der zur Platzierung im oder vor dem Aneurysma bestimmt ist, und einen Übergangsabschnitt zwischen dem Fixierabschnitt und dem distalen Abschnitt aufweist, wobei das Implantat aus miteinander verbundenen oder sich kreuzenden Filamenten aufgebaut ist und im Übergangsabschnitt ein oder mehrere vom Fixierabschnitt oder distalen Abschnitt ausgehende Filamente zentral zusammenkommen. Ein solches Implantat wird mit Hilfe eines Katheters und Führungsdrahts an den Implantationsort gebracht und dort dauerhaft implantiert. Entsprechend betrifft die Erfindung auch ein solches Implantat, implantationsfertig angekoppelt an einen Führungsdraht.

Darüber hinaus betrifft die Offenbarung ein Verfahren zur Einbringung des Implantats.

Arteriovenöse Fehlbildungen können in einem Patienten zu erheblichen Beeinträchtigungen und Gefährdungen bis hin zum Tode führen. Dies gilt besonders für Aneurysmen, insbesondere dann, wenn sie im zerebralen Bereich auftreten. In der Regel versucht man derartige Fehlbildungen durch Implantate zu verschließen. Solche Implantate werden zumeist auf endovaskulärem Weg mit Hilfe von Kathetern gesetzt.

Insbesondere bei zerebralen Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma ausfüllt und letztlich verschließt. Diese Behandlungsmethode ist allerdings nur bei Aneurysmen geeignet, die über einen relativ engen Zugang zum Gefäßsystem verfügen, sogenannten Beerenaneurysmen. Bei Aussackungen von Blutgefäßen, die über einen weiten Zugang zum Gefäß verfügen, drohen die implantierten Spiralen wieder ausgeschwemmt zu werden. Diese können in andere Bereiche des Gefäßsystems gelangen und dort Schäden herbeiführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, werden bereits bei einigen Formen von Aneurysmen eingesetzt.

Gefäßverzweigungen, insbesondere Gefäßbifurkationen, sind ein relativ häufiges Phänomen. Das durch eine Arterie im Bereich einer Gabelung auf die Stirnwand aufprallende Blut führt - im Fall einer Schwächung der Gefäßwandung - schnell zu einer Aussackung, die sich dann rasch erweitert. Solche Bifurkationsaneurysmen haben häufig einen weiten Hals, der eine Therapie nur mit Okklusionsspiralen unmöglich macht.

Vaskuläre Implantate, die geeignet sind, im Bereich einer Gefäßverzweigung eine "Vergitterung" des Aneurysmaeingangs herbeizuführen, werden beispielsweise in den internationalen Patentanmeldungen WO 2012/113554 A1 oder WO 2014/029835 A1 offenbart. Mit nach dem Setzen des Implantats eingebrachten Okklusionsspiralen kann dann das Aneurysma stillgelegt werden. Möglich ist auch, dass das Implantat selber das Aneurysma in ausreichendem Maße vom Blutstrom abkoppelt. Hierzu kann beispielsweise das Implantat eine Membran aufweisen, die im Bereich des Aneurysmahalses oder vor dem Aneurysmahals platziert wird. Ggf. kann auch allein mit Filamenten, typischerweise Drähten geringen Durchmessers, der Blutstrom zum Aneurysma so weit reduziert werden, dass auf die zusätzliche Einbringung von Okklusionsspiralen oder anderen Okklusionsmitteln in das Aneurysma verzichtet werden kann.

Aus dem Stand der Technik bekannte Implantate weisen einen proximalen Abschnitt auf, der der Fixierung des Implantats im Trägergefäß dient und im Wesentlichen die Form eines herkömmlichen Stents aufweist. Am distalen Ende des Implantats ist ein distaler Abschnitt vorgesehen, der im oder vor dem Aneurysma platziert werden soll, um das Aneurysma vom Blutfluss abzuschneiden und/oder in das Aneurysma eingebrachte Okklusionsmittel daran zu hindern, aus dem Aneurysma in das Blutgefäß auszutreten. Zwischen dem proximalen und dem distalen Abschnitt kann ein Zwischenabschnitt vorgesehen sein, der beispielsweise über eine relativ geringe Dichte der Filamente verfügt, um den Blutfluss in abzweigende Blutgefäße nicht oder nur geringfügig zu behindern.

Als problematisch hat sich bei der Platzierung von für Bifurkationsaneurysmen geeigneten Implantaten herausgestellt, dass viele Aneurysmen eine unregelmäßige Form aufweisen, beispielsweise nicht symmetrisch sind oder zu einer Seite abkippen. Insofern ist eine hohe Flexibilität des distalen Abschnitts des Implantats, das im oder vor dem Aneurysma zu liegen kommen soll, von Vorteil. Entsprechend wird beispielsweise in der WO 2012/078678 A1 vorgeschlagen, einen sehr schmalen Zwischenabschnitt vorzusehen, der aus ein oder mehreren Filamenten aufgebaut ist. Eine weitere Vorrichtung zum Verschluss von Aneurysmen mit einem Embolisationselement wird in der US 2007/0270902 A1 beschrieben. Hier ist ein stentartiges Verankerungselement vorgesehen, das über ein sehr schmales Verbindungselement mit dem Embolisationselement verbunden ist. Das Embolisationselement ist trichterförmig ausgebildet.

Auch dies hat sich jedoch als nicht ausreichend für manche Aneurysmen herausgestellt, da die im Zwischenabschnitt vorliegenden Drähte entweder zu biegesteif sind, um eine gute Anpassung an das Aneurysma zu erlauben, oder dazu tendieren, sich seitlich abzuspreizen, wodurch wiederum der Blutfluss in angrenzende Blutgefäße behindert oder Blutgefäße verletzt werden können.

Es stellt sich somit die Aufgabe, ein Implantat zum Einsatz bei der Okkludierung von Aneurysmen in Blutgefäßen im Bereich von Gefäßverzweigungen zur Verfügung zu stellen, bei dem der distale Abschnitt sich besonders flexibel an die Form des jeweiligen Aneurysmas anpassen kann.

Diese Aufgabe wird gelöst durch ein Implantat zum Einsatz bei der Okkludierung von Aneurysmen in Blutgefäßen im Bereich von Gefäßverzweigungen, insbesondere Bifurkationsaneurysmen, wobei das Implantat in einem expandierten Zustand, in dem es im Blutgefäß implantiert wird, und in einem kontrahierten Zustand vorliegt, in dem es durch das Blutgefäß bewegbar ist, wobei das Implantat einen proximalen Fixierabschnitt, mit dem das Implantat an einer Gefäßwand des Blutgefäßes fixierbar ist, einen distalen Abschnitt, in dem das Implantat gegenüber dem Fixierabschnitt radial erweitert ist und der zur Platzierung im oder vor dem Aneurysma bestimmt ist, und einen Übergangsabschnitt zwischen dem Fixierabschnitt und dem distalen Abschnitt aufweist, wobei das Implantat aus miteinander verbundenen oder sich kreuzenden Filamenten aufgebaut ist und im Übergangsabschnitt ein oder mehrere vom Fixierabschnitt oder distalen Abschnitt ausgehende Filamente zentral zusammenkommen, wobei die Filamente im Übergangsabschnitt zumindest bereichsweise durch eine Hülse verlaufen und ein oder mehrere Filamente im Übergangsabschnitt eine Öse aufweisen, wobei die Hülse zumindest abschnittsweise durch die Öse verläuft und die Öse die Hülse in ihrer Beweglichkeit in Längsrichtung des Implantats einschränkt.

Erfindungsgemäß läuft das Implantat in Längsrichtung betrachtet vom Fixierabschnitt ausgehend im Übergangsabschnitt eng zusammen und erweitert sich wiederum im distalen Abschnitt. Der Querschnitt des Implantats in Längsrichtung ist somit im Übergangsabschnitt erheblich kleiner als im distalen Abschnitt oder im Fixierabschnitt. Im Übergangsabschnitt liegen lediglich ein oder mehrere Filamente eng nebeneinander. Diese Filamente verlaufen zumindest bereichsweise durch eine Hülse, die die Filamente im Übergangsabschnitt zusammenhält. Unter "bereichsweise" wird in diesem Zusammenhang verstanden, dass die Filamente im Übergangsabschnitt nicht unbedingt über ihre gesamte Länge durch die Hülse verlaufen müssen; es ist ausreichend, wenn ein Teil der Länge der Filamente im Übergangsabschnitt von der Hülse umgeben ist. Mit anderen Worten kann die Hülse kürzer sein als der Übergangsabschnitt und die diesen ausbildenden Filamente.

Bei den Filamenten des Übergangsabschnitts kann es sich um gesonderte Filamente handeln, die einerseits am distalen Abschnitt, andererseits am Fixierabschnitt festgelegt sind. Bevorzugt ist allerdings, dass es sich um dieselben Filamente handelt, die auch den Fixierabschnitt und den distalen Abschnitt ausbilden und lediglich im Bereich des Übergangsabschnitts zentral zusammenlaufen.

Dadurch, dass die Filamente im Übergangsabschnitt durch eine die Filamente umgebende Hülse zusammengehalten werden, bleiben die Filamente in gewissem Rahmen relativ zueinander beweglich, können jedoch gleichwohl nicht über das vorgesehene Maß hinaus radial expandieren. Das Risiko, dass sich eines oder mehrere Filamente im Übergangsabschnitt radial abspreizen, ist somit ausgeräumt. Gleichwohl ist das Implantat um den Übergangsabschnitt sehr flexibel und kann sich daher gut anpassen an die Gestalt der Blutgefäße und des Aneurysmas. Andere Möglichkeiten der Fixierung der Filamente aneinander wären zwar möglicherweise ebenfalls in der Lage, das Risiko der radialen Abspreizung von Filamenten zu minimieren, würden jedoch die Beweglichkeit der Filamente und damit auch die Flexibilität des Implantats stark einschränken. Besondere Bedeutung hat die Erfindung für stark asymmetrische Aneurysmen und Aneurysmen, die zu einer Seite abkippen.

Durch die besonders dünne Ausgestaltung des Übergangsabschnitts wird auch gewährleistet, dass der Blutfluss zu abzweigenden Gefäßen nicht oder kaum beeinträchtigt ist. Mit anderen Worten sorgt der Fixierabschnitt für eine Fixierung des Implantats im Trägergefäß, der Übergangsabschnitt für eine ausreichende Flexibilität sowie den ungehinderten Blutstrom von und zu abzweigenden Gefäßen und der distale Abschnitt für den Verschluss des Aneurysmas, wobei der distale Abschnitt entweder unmittelbar den Blutfluss in das Aneurysma minimieren kann oder dafür sorgt, dass in das Aneurysma eingeführte Okklusionsmittel im Aneurysma verbleiben.

Der distale Abschnitt kann, je nach Form des Aneurysmas und Gestalt des Implantats im Aneurysma selbst platziert werden oder auch vor dem Aneurysma, d.h. vor dem Aneurysmahals auf der Seite des Trägergefäßes. Als Platzierung im Aneurysma wird in diesem Zusammenhang auch die Platzierung im Aneurysmahals angesehen, der zum Aneurysma gehört. In der Regel wird der distale Abschnitt im Eintrittsbereich des Aneurysmas positioniert.

Der Begriff "Hülse" ist erfindungsgemäß weit zu verstehen, d.h. es kann sich um ein kurzes Rohr, aber auch um sonstige Gestaltungen handeln, vorausgesetzt, die Hülse weist einen inneren Hohlraum auf, durch den die Filamente verlaufen können. Die Hülse kann beispielsweise die Form einer Manschette haben. Insbesondere kann es sich bei der Hülse auch um eine Drahtwendel handeln, die einen inneren Hohlraum aufweist.

Wichtig ist, dass die Hülse bei kontrahiertem Implantat nicht verrutscht und womöglich in den Bereich des Fixierabschnitts oder des distalen Abschnitts gelangt. In diesem Fall könnte es nämlich dazu kommen, dass die Hülse die Expansion des Implantats nach Freisetzung behindert. Sofern ein entsprechendes Verrutschen der Hülse nicht durch die Dimensionen der einzelnen Abschnitte des Implantats ohnehin ausgeschlossen ist, kann es daher sinnvoll sein, proximal und/oder distal der Hülse Stopper vorzusehen, die ein Verschieben oder Verrutschen der Hülse über die Stopper hinaus verhindern. Bei den Stoppern kann es sich beispielsweise um radiale Erweiterungen im Übergangsabschnitt handeln.

Um ein Verrutschen der Hülse zu verhindern, sind ein oder mehrere Filamente im Übergangsabschnitt mit einer Öse versehen, wobei die Hülse jedenfalls abschnittsweise durch die Öse verläuft, sodass die Hülse in ihrer Beweglichkeit in Längsrichtung des Implantats eingeschränkt wird. Die Öse kann z. B. dadurch geschaffen werden, dass ein oder mehrere Filamente im Übergangsabschnitt einen größeren Querschnitt aufweisen und im Bereich des größeren Querschnitts eine Öffnung im Filament vorhanden ist. Die Öffnung ist typischerweise länglich, sodass eine gewisse Längsverschiebbarkeit der Hülse zwecks Gewährleistung einer ausreichenden Flexibilität gegeben ist, die Längsverschiebbarkeit jedoch durch das distale und das proximale Ende der Öse begrenzt wird. Die Öffnung verläuft normalerweise im Wesentlichen orthogonal zur Längsachse des Implantats. In der Regel weist das betreffende Filament die Öse auf der Außenseite auf, sodass das Filament im Übrigen von der Hülse umgeben wird, wobei die Hülse durch die Öse verläuft. Denkbar ist jedoch auch der umgekehrte Fall, bei dem die Öse vom betreffenden Filament aus in Richtung Zentrum des Übergangsabschnitts weist; auch in diesem Fall verläuft die Hülse durch die Öse, der übrige Bereich des Filaments liegt jedoch außerhalb der Hülse. Ein Implantat zur Behandlung von Aneurysmen im Bereich von Bifurkationen mit einer Einschnürung in einem Übergangsabschnitt, die von einem Markerband umgeben ist, wird in der US 2006/0064151 A1 offenbart, besondere Maßnahmen, um eine Verschiebung des Markerbandes zu verhindern, sind hier jedoch nicht vorgesehen.

Besonders vorteilhaft ist, wenn die Hülse zumindest teilweise aus einem röntgensichtbaren Material gefertigt ist. Dies erlaubt, typischerweise zusammen mit weiteren röntgensichtbaren Markierungen am Implantat, eine Visualisierung und damit Kontrolle des Implantationsvorgangs. Bevorzugt ist in diesem Zusammenhang eine Fertigung der Hülse aus Platin, Platin-Legierungen wie Platin-Iridium oder Gold. Möglich ist eine Fertigung der Hülse, beispielsweise der Drahtwendel aus dem entsprechenden Material, denkbar ist jedoch auch ein Überzug wie z. B. in Form einer Goldbeschichtung.

Um eine gewisse Beweglichkeit der Filamente zueinander zu erhalten, ist es bevorzugt, wenn die Filamente im Übergangsabschnitt parallel zueinander verlaufen. Denkbar ist auch eine leichte Verdrillung der Filamente, diese sollte aber nicht zu steif ausfallen, um die Anpassungsfähigkeit des Implantats nicht zu gefährden.

Die Begriffe "proximal" und "distal" sind so zu verstehen, dass sie beim Einsetzen des Implantats zum behandelnden Arzt weisende Teile des Implantats bezeichnen (proximal) bzw. vom behandelnden Arzt weg weisende Teile (distal). Das Implantat wird somit typischerweise durch einen Katheter in distaler Richtung vorgeschoben. Der Begriff "axial" bezieht sich auf die von proximal nach distal verlaufende Längsachse des Implantats, der Begriff "radial" auf hierzu senkrechte Ebenen.

Bei dem erfindungsgemäßen Implantat kann es sich um ein Implantat mit einer Maschenstruktur handeln, die aus einem Geflecht einzelner Drähte bestehen kann, eine aus einem Rohr geschnittene Maschenstruktur aufweisen kann oder eine Kombination von beidem ist. Insoweit handelt es sich bei dem Implantat weitgehend um einen Stent oder ein stentähnliches Gebilde, das sich durch die besondere Art seines Einsatzes und Aufbaus auszeichnet. Die Ähnlichkeit mit einem Stent gilt insbesondere für den Fixierabschnitt, während der distale Abschnitt radial nach außen erweitert ist und beispielsweise nach außen weisende Bögen aufweisen kann. Im Falle der Flechtung aus einzelnen Filamenten wird für den Fixierabschnitt eine Zahl von 3 bis 24 Filamenten bevorzugt.

Bei den das Implantat ausbildenden Filamenten handelt es sich zumeist um Drähte oder Stege, insbesondere aus Metall. Besonders bevorzugt ist die Verwendung von Formgedächtnismetallen wie Nickel-Titan-Legierungen, die auch unter dem Namen Nitinol bekannt sind. Auch ternäre Nickel-Titan-Legierungen können zum Einsatz kommen. Ebenso möglich ist die Verwendung anderer herkömmlicher Stentmaterialien wie medizinischer Stahl oder Kobalt-Chrom-Legierungen.

Die Filamente können einen runden, ovalen, quadratischen oder rechteckigen Querschnitt aufweisen. Möglich ist auch die Verwendung von flachen Filamenten in Form dünner Streifen, insbesondere Metallstreifen.

Am proximalen Ende des Fixierabschnitts können ein oder mehrere Kupplungselemente angeordnet sein. Insbesondere kann der Fixierabschnitt nach proximal in die Kupplungselemente übergehen. Die Kupplungselemente befinden sich vorzugsweise an der Peripherie, d. h. exzentrisch auf dem Umfang des Implantats in seiner expandierten Form und kommen im implantierten Zustand, wenn das Implantat in seiner expandierten Form vorliegt, an der Gefäßwandung zu liegen. Die Kupplungselemente dienen der Verbindung mit einer Einführhilfe, insbesondere einem Führungsdraht.

Die exzentrische Anordnung des oder der Kupplungselemente am proximalen Ende des Fixierabschnitts erleichtert bei Fehlplatzierungen das Rückziehen des Implantats in den Platzierungskatheter. Bevorzugt sind Ausführungsformen mit ein bis drei Kupplungselementen. Vorzugsweise handelt es sich bei den Kupplungselementen um Kupplungsdrähte. Das Vorsehen mehrerer Kupplungselemente verbessert die Rückziehbarkeit, insbesondere dann, wenn der Fixierungsabschnitt relativ kurz ist. Die lösbare Verbindung der Kupplungselemente mit der Einführhilfe kann unterschiedlich ausgestaltet sein, bevorzugt ist eine elektrolytisch ablösbare Verbindung wie aus dem Stand der Technik bekannt. Ebenso denkbar sind aber auch thermische oder mechanische Ablösesysteme.

Die Kupplungselemente, insbesondere die Kupplungsdrähte, bzw. das proximale Ende des Implantats (ohne Einführhilfe) können im expandierten, von äußeren Zwängen freien Zustand zur Längsachse des Implantats einen Winkel zwischen 0° und +60° ausbilden, wobei ein positiver Winkel für ein nach außen weisendes proximales Ende steht. Bevorzugt ist ein Bereich zwischen +10° und +30°, wobei der optimale Winkel von der Gestalt des Gefäßes abhängt. Ein solcher positiver Winkel erleichtert die optimale Expansion des Implantats und das Anlegen des proximalen Endes an das Trägergefäß; das Hineinragen des proximalen Endes in das Gefäßlumen, das den Blutfluss oder das Einführen eines weiteren Mikrokatheters stören könnte, wird wirkungsvoll verhindert. Vorzugsweise ist das proximale Ende des Implantats atraumatisch ausgebildet, um eine Verletzung der Gefäßwand auszuschließen. Die Ausbildung des Winkels ist erfindungsgemäß so zu verstehen, dass der Winkel nicht im kontrahierten Zustand vorliegen muss, d. h. es ist ausreichend, dem Implantat eine entsprechende Verformung im expandierten Zustand aufzuprägen. Hier bietet sich insbesondere die Verwendung von Formgedächtnismaterialien an.

Mit dem Fixierabschnitt stützt sich das Implantat an der Gefäßwand des Blutgefäßes ab, in dem das Implantat implantiert wird, und wird dadurch fixiert. In diesem Bereich ist das Gefäß nicht geschädigt und geeignet, den einer Stentwandung ähnelnden Fixierabschnitt zu tragen. Bei selbstexpandierenden Implantaten legt sich der Fixierabschnitt nach der Freisetzung aus dem Katheter selbständig an die Gefäßwandung an, bei ballonplatzierbaren Implantaten wird das Implantat in diesem Bereich durch einen Platzierungsballon aufgeweitet und gegen die Gefäßwand gepresst. Bevorzugt sind selbstexpandierende Implantate.

Der distale Abschnitt ist gegenüber dem Fixierabschnitt und noch stärker gegenüber dem Übergangsabschnitt radial nach außen erweitert. Er dient der Platzierung im Aneurysma selbst oder im Bereich des Zugangs zum Aneurysma, d. h. am Aneurysmahals und verschließt diesen bzw. hindert in das Aneurysma eingebrachte Okklusionsmittel am Austritt. Entscheidend ist, dass letztlich im Aneurysma eine Blutgerinnung einsetzt. Auf der einen Seite muss die Oberflächenabdeckung hinreichend groß sein, um in das Aneurysma eingebrachte Okklusionsmittel daran zu hindern, aus dem Aneurysma zu entweichen, oder durch ausreichendes Material eine dichte Oberfläche zu schaffen, auf der anderen Seite muss eine hinreichend große Flexibilität des Implantats erhalten bleiben, um es im Bereich des Bifurkationsaneurysmas einbringen zu können.

Der distale Abschnitt kann im expandierten Zustand nach radial außen weisende Streben, Schlaufen oder Bögen aufweisen. Diese dienen der Verankerung des Implantats im oder vor dem Aneurysma. Häufig zeigt daher das Implantat von distal betrachtet im distalen Abschnitt eine Blütenform. In der Regel sind wenigstens zwei Streben/Schlaufen/Bögen vorgesehen, insbesondere drei Streben/Schlaufen/Bögen oder mehr. Typischerweise beträgt die Zahl der Streben/Schlaufen/Bögen 1 bis 24, bevorzugt sind 2 bis 8. Bei den Streben, Schlaufen oder Bögen kann es sich um entsprechend geformte Drahtelemente handeln, sie können aber auch, soweit das Implantat aus einem Rohr geschnitten ist, entsprechend durch Laserschneiden des gleichen Rohrs, in der Regel gefolgt von einer Wärmebehandlung, erzeugt sein. Ein Anfügen der Streben, Schlaufen oder Bögen ist beispielsweise mittels Laserschweißen möglich. Im Falle von Schlaufen oder Bögen handelt es sich vorzugsweise um vom Übergangsabschnitt ausgehende, eine Biegung ausbildende und zurück verlaufende Drahtelemente, wobei grundsätzlich beliebig komplexe Formen für die Schlaufen/Bögen denkbar sind. Insbesondere kann es sich auch um dreidimensionale Objekte handeln, je nachdem, wie die Schlaufen oder Bögen verlaufen. Die Schlaufen bzw. Bögen sollten weitgehend atraumatisch sein und die empfindliche Gefäßwand des Aneurysmas nicht verletzen. Möglich ist aber auch der Einsatz sonstiger Filamente oder Streben, durch die eine radiale Erweiterung des distalen Abschnitts gegenüber dem Fixierabschnitt und dem Übergangsabschnitt herbeigeführt wird. Die Erweiterung kann beispielsweise trompetenförmig, korbförmig, blütenförmig oder in Form eines Geflechts vorliegen. Nach außen abstehende Streben sind vorzugsweise nach radial innen konzentrisch ausgerichtet. Gleichzeitig können die Streben in Richtung distal ragen. Beispielsweise können jeweils zwei oder mehr Streben von einem gemeinsamen Verbindungspunkt ausgehen.

Der Winkel, den die Streben/Schlaufen/Bögen zur Längsachse des Implantats im implantierten Zustand ausbilden, liegt zwischen -45° und +175°, wobei ein positiver Winkel für nach radial außen und ein negativer Winkel für nach radial innen weisende Streben/Schlaufen/Bögen steht. Im Falle von verhältnismäßig regelmäßigen Bifurkationsaneurysmen liegt der Winkel bevorzugt bei +45° bis +90°, teilweise treten jedoch auch Aneurysmen auf, die eine unregelmäßige Form aufweisen, insbesondere stark asymmetrisch sind. In solchen Fällen kann es sinnvoll sein, stark abweichende Winkel der Streben/Schlaufen/Bögen zum Einsatz zu bringen. Beispielsweise kann es sinnvoll sein, den Winkel sehr groß zu wählen, wenn die Wandung in einem Bereich des Aneurysmas stark in Richtung des zuführenden Gefäßes ausgewölbt ist. In solchen Fällen sind Winkel > 90° denkbar. In anderen Fällen kann es zweckmäßig sein, einen Teil der Streben/Schlaufen/Bögen nach innen weisen zu lassen, d. h. negative Winkel zu wählen, um sich an die Wandung des Aneurysmas anzupassen. Die erfindungsgemäß durch den sehr schmalen Übergangsabschnitt mit Hülse herbeigeführte Flexibilität sorgt allerdings dafür, dass sich die Streben/Schlaufen/Bögen auch ohne besonders große oder kleine voreingestellte Winkel gut an die Form des Aneurysmas anpassen können. Die Winkel können variieren, beispielsweise kann es bei einem asymmetrischen Aneurysma sinnvoll sein, einige Schlaufen mit Winkeln > 90° vorzusehen, während andere Schlaufen herkömmliche Winkel im Bereich zwischen 45° und 90° aufweisen. Wichtig ist, dass die genannten Winkel nach Implantierung ausgebildet werden, auch ein Implantat, bei dem im Zustand vor der Implantierung, etwa durch äußere Zwänge, sich die angegebenen Winkel noch nicht ausgebildet haben, ist daher als erfindungsgemäß anzusehen.

Winkel, die die Streben/Schlaufen/Bögen zur Längsachse des Implantats ausbilden, können z. B. zwischen 45° und 90°, -45° und 0°, 90° und 135° oder 135° und 175° liegen.

Die Streben/Schlaufen/Bögen im distalen Abschnitt können Fortsetzungen der das übrige Implantat ausbildenden Filamente sein, es kann sich aber auch um gesonderte Filamente handeln, die im distalen Bereich des übrigen Implantatkörpers, d. h. am distalen Ende des Übergangsabschnitts festgelegt sind, beispielsweise durch Laserverschweißen. Dabei kann jede Strebe/jede Schlaufe oder jeder Bogen des distalen Abschnitts an einem oder mehreren Verbindungspunkten mit dem weiteren Implantatkörper verbunden sein, insbesondere können auch nur ein oder zwei Verbindungspunkte pro Schlaufe/Strebe/Bogen vorgesehen sein.

Alternativ zur Ausbildung des distalen Abschnitts aus Schlaufen oder Bögen kann der distale Abschnitt auch kugelförmig, pilzförmig, ankerförmig oder ellipsoidförmig erweitert sein. Bei den genannten Formen handelt es sich um Alternativen, durch die ebenfalls ein radial erweiterter distaler Abschnitt ausgebildet werden kann. Ein kugelförmiger Abschnitt kann sich beispielsweise gut an die Innenwandungen des Aneurysmas anschmiegen, da ein regelmäßiges Bifurkationsaneurysma häufig im Wesentlichen die Form einer Kugel aufweist. Dabei sei erwähnt, dass als Kugelform im Sinne der Erfindung nicht nur exakte Kugeln entsprechend der geometrischen Definition verstanden werden, vielmehr werden auch hiervon abweichende, runde, dreidimensionale Formen erfindungsgemäß als Kugeln angesehen. In einigen Fällen ähnelt die Form des Abschnitts auch einem Ellipsoid, wobei auch hier gilt, dass das Vorliegen eines exakten Rotationsellipsoids nicht erforderlich ist, um als ellipsoidförmig im Sinne der Erfindung zu gelten. Weitere Möglichkeiten sind pilz- oder ankerförmige Abschnitte, die sich insbesondere bei unregelmäßigen Aneurysmen eignen, beispielsweise wenn die Wandung in einem Bereich des Aneurysmas stark in Richtung des zuführenden Gefäßes ausgewölbt ist. Dies wird dadurch gewährleistet, dass bei einer Pilz- oder Ankerform einige Bereiche des Abschnitts in proximaler Richtung verlaufen. Dabei sei klargestellt, dass ein pilz- oder ankerförmiger Abschnitt selbst ebenfalls asymmetrisch sein, beispielsweise nur auf einer Seite in proximaler Richtung verlaufende Bereiche aufweisen kann. Der distale Abschnitt kann lasergeschnitten oder auch geflochten sein, wobei bevorzugt 8 bis 128 Filamente zum Einsatz kommen.

Im distalen Abschnitt kann zentral ein Bereich vorgesehen sein, der der Blockade des Aneurysmas dient, nämlich der Verhinderung des Austritts von Okklusionsmitteln und/oder der Abkopplung des distalen Abschnitts vom Blutfluss. Die hierfür vorgesehenen Elemente werden als Trennelemente bezeichnet. Der Bereich kann einerseits durch das Einziehen von Fasern, Fäden, dünnen Drähten, einer Membran oder dergleichen Trennelemente ausgebildet werden, kann aber auch integraler Teil des Implantats in dem Sinne sein, dass es sich um aus dem Ausgangsrohr herausgeschnittene und entsprechend umgeformte Trennelemente oder um aus einem Drahtgeflecht gebildete Trennelemente, etwa Schlaufen oder Stege, handelt. Im Falle von Schlaufen oder Stegen weisen diese radial nach innen in das Lumen des Implantats, im Gegensatz zu den zuvor beschriebenen Schlaufen des distalen Abschnitts, die zumindest größtenteils nach außen weisen. Damit die nach innen weisenden Schlaufen/Stege sich nicht gegenseitig behindern, kann es sinnvoll sein, diese asymmetrisch auszugestalten. Die Zahl kann variieren, abhängig von der Struktur des Implantats.

Als Trennelemente ausgebildete Fäden können aus einem Polymermaterial hergestellt sein, beispielsweise einem Polyamid wie Nylon (Polyhexamethylenadipinsäureamid). Ebenso möglich ist die Herstellung aus Metall, wobei Formgedächtnislegierungen bevorzugt sind, insbesondere Nickel-Titan-Legierungen wie Nitinol.

Eine weitere Möglichkeit besteht darin, als Trennelement eine Membran vorzusehen, die weitgehend oder vollständig undurchlässig für Blut ist und das Aneurysma auf diese Weise vom Blutstrom abkoppelt. Sofern eine weitgehend vollständige Abkopplung vom Blutstrom erreicht wird, ist eine Einbringung von Okklusionsmitteln in das Aneurysma u. U. entbehrlich, d. h. das Trennelement dient in diesem Fall nicht zum Zurückhalten von Okklusionsmitteln. Die Membran kann an den Filamenten fixiert und/oder auf ein Geflecht aus Fäden oder Drähten aufgespannt sein, bspw. können Fäden oder Drähte eine Struktur bilden, über oder auf die die Membran aufgespannt ist. Zusätzlich sind weitere, z. B. kreuzförmig verlaufende Fäden/Drähte denkbar, die ein Fadenkreuz ausbilden. Fäden oder Drähte sind jedoch nicht unbedingt erforderlich, auch ein Überspannen des zentralen Bereichs des distalen Abschnitts ohne zusätzliche Fäden/Drähte ist möglich.

Ein Vorteil der Verwendung einer Membran als Trennelement ist darin zu sehen, dass diese sich bei der Platzierung des Implantats im Katheter eng nach distal oder proximal zusammenlegt, so dass ein Implantat mit im expandierten Zustand weitgehend dichtem Trennelement zur Verfügung gestellt wird, das sich im kontrahierten Zustand auch durch enge Blutgefäße gut hindurchführen lässt. Im Übrigen bleibt der Aufbau des Implantats im Vergleich zu einem Implantat ohne Trennelement weitgehend unverändert.

Auch in den Fällen, in denen als Trennelement eine Membran vorgesehen ist, kann es jedoch von Vorteil sein, zusätzlich Okklusionsmittel in das Aneurysma einzubringen. Aus diesem Grund kann es sinnvoll sein, eine Membran mit einer oder mehreren Ausnehmungen zu verwenden, so dass durch die Ausnehmung ein Einbringen von Okklusionsmitteln, insbesondere Coils möglich ist. Die Ausnehmung sollte eine Größe aufweisen, dass ein Katheter durch die Ausnehmung in den Bereich des Aneurysmas vorgeschoben werden kann, wobei durch den Katheter die Okklusionsmittel eingebracht werden. Andererseits sollte der Aneurysmahals so weit abgedeckt sein, dass die Okklusionsmittel das Aneurysma nicht unkontrolliert verlassen können, wobei ggf. den Bereich der Membran überspannende Fäden/Drähte eine zusätzliche Rückhaltefunktion erfüllen. Selbstverständlich dürfen in diesem Fall die Fäden bzw. Drähte nur so dicht verlaufen, dass ein Hindurchführen eines Katheters und ein Einbringen von Okklusionsmitteln weiterhin möglich ist.

Um Okklusionsmittel in das Aneurysma einzubringen, kann die Membran auch partiell durchstoßbar ausgebildet werden, wobei zum Durchstoßen typischerweise ein Mikrokatheter oder ein Führungsdraht verwendet wird. Durch die so geschaffene Öffnung wird sodann ein Mikrokatheter geführt, durch den die Okklusionsmittel eingeschoben werden. Die Membran sollte so beschaffen sein, dass sie auch nach Durchstoßen partiell erhalten bleibt, so dass die Okklusionsmittel weiterhin durch die Membran am Wiederaustritt gehindert werden. Beispielsweise können als zusätzliche Trennelemente vorgesehene Fäden oder Drähte, die in der Form eines Fadenkreuzes aufgespannt sein können, dafür sorgen, dass lediglich ein Segment der Membran beim Durchstoßen eine Öffnung ausbildet, während die übrigen Segmente von der Membran abgedeckt bleiben, da die Randbereiche der Membran von den Fäden/Drähten stabilisiert und vor einem Einreißen geschützt werden. Bei der als Trennelement vorgesehenen Membran kann es sich um eine einzelne Membran handeln, die nur partiell durchstoßen wird, oder auch um mehrere kleine Membranen.

Statt oder zusätzlich zum Vorsehen einer Membran als Trennelement kann es sinnvoll sein, Membranen im Inneren der (Draht)schlaufen oder Bögen des distalen Abschnitts vorzusehen. Membranen können auch zwischen Streben des distalen Abschnitts vorgesehen sein. Auch kugel-, pilz-, anker- oder ellipsoidförmig ausgebildete distale Abschnitte können mit einer Membran bespannt sein. Die Membranen können zum einen bei Platzierung vor oder im Eintrittsbereich des Aneurysmas der Umlenkung des Blutstromes in abzweigende Gefäße, zum anderen der Unterbindung des Blutstromes in das Aneurysma dienen.

Die Membran muss nicht auf Trennelement und das Innere der Schlaufen/Bögen beschränkt sein, sondern kann insgesamt den distalen Abschnitt überspannen, wobei die Streben, Schlaufen oder Bögen der Fixierung der Membran dienen können. Beispielsweise können Membranen in den Zwischenräumen zwischen den Streben, Schlaufen oder Bögen vorgesehen sein.

Auch wenn der distale Abschnitt ganz oder teilweise von anderen Filamenten als Schlaufen gebildet wird, ist es möglich, hier Membranen vorzusehen. Beispielsweise können eine oder mehrere Membranen von radial nach außen abstehenden Streben aufgespannt werden. In diesem Fall ähnelt der Aufbau dem eines Schirms, d. h. beim Expandieren des distalen Abschnitts spannen die sich entfaltenden Streben eine durchgehende oder mehrere Membranen zwischen sich auf. Durch das Vorsehen von mehreren Streben und einer entsprechenden Zahl an Strebenenden wird erreicht, dass die von der Membran abgedeckte Fläche größer und kreisähnlicher wird und weniger große Zwischenräume aufweist.

Zur Abgrenzung und Verstärkung der Membran können auch zwischen den einzelnen Streben/Schlaufen/Bögen gespannte Fäden dienen, d. h. die Membranen werden zumindest teilweise seitlich von einem oder mehreren Fäden begrenzt, die die Streben/Schlaufen/Bögen miteinander verbinden. Dabei muss die Begrenzung der jeweiligen Membran nicht in jeder Richtung über einen Faden erfolgen, hierzu können auch teilweise die Streben/Schlaufen/Bögen selbst dienen. Beispielsweise kann der äußere Rand der Membran, der häufig auch weiter distal liegt, durch Fäden, der innere Rand durch Streben/Schlaufen/Bögen umrandet sein. Im Vergleich zu einer Membran ohne seitliche Begrenzung wird insbesondere ein zusätzlicher Schutz der Membran gegen Beschädigungen und Risse erreicht. Die Fäden werden bevorzugt aus einem Polyamid wie Nylon gefertigt.

Die Membran (unabhängig davon, ob als Trennelement verwendet oder in anderen Bereichen des distalen Abschnitts angeordnet) kann aus einem Polymermaterial wie Polytetrafluorethylen, Polyester, Polyamiden, Polyurethanen oder Polyolefinen hergestellt sein. Besonders bevorzugt sind Polycarbonaturethane. Wünschenswert ist insbesondere eine integrale Verbindung der Membran mit ggf. als weitere Trennelemente vorgesehenen Fäden oder Drähten. Diese kann durch eine Tauch- oder Sprühbeschichtung der Fäden/Drähte herbeigeführt werden.

Vorzugsweise wird die Membran durch Elektrospinnen erzeugt. Hierbei werden Fibrillen bzw. Fasern aus einer Polymerlösung mit Hilfe von elektrischem Strom auf einem Substrat abgeschieden. Bei der Abscheidung verkleben die Fibrillen zu einem Vlies. In der Regel haben die Fibrillen einen Durchmesser von 100 bis 3000 nm. Durch Elektrospinnen gewonnene Membranen sind sehr gleichmäßig ausgebildet und können eine Grundstruktur aus Fäden oder Drähten in sich einzuschließen. Die Membran ist zäh und mechanisch belastbar und kann mechanisch durchstoßen werden, ohne dass die Öffnung zum Ansatzpunkt für weitergehende Risse wird. Die Dicke der Fibrillen wie auch der Grad der Porosität kann durch Auswahl der Verfahrensparameter gesteuert werden. Im Zusammenhang mit der Schaffung der Membran und den dafür geeigneten Materialien wird insbesondere auf die WO 2008/049386 A1, die DE 28 06 030 A1 und die darin genannte Literatur hingewiesen.

Vorteilhaft ist auch ein Implantat, bei dem als Trennelement eine an der Innenseite des Implantats anliegende Membran verwendet wird, wobei diese Membran wiederum mit weiteren äußeren Membranabschnitten fest verbunden ist, die die einzelnen Schlaufen oder Bögen des distalen Abschnitts ausfüllen. Eine solche Membranstruktur lässt sich über Elektrospinnen erzeugen. Die inneren und äußeren Membranabschnitte sind in diesem Fall teilweise verbunden; dort, wo der innere Membranabschnitt keine Verbindung mit dem äußeren Membranabschnitt aufweist, zieht er sich ähnlich einem Nylonstrumpf zusammen, sodass sich eine Öffnung für die Einbringung von Okklusionsmitteln ergibt.

Statt durch Elektrospinnen kann die Membran auch über einen Tauchprozess hergestellt werden.

Eine als Trennelement dienende Membran muss nicht in jedem Fall in einer Ebene orthogonal zur Längsachse des Implantats verlaufen, sondern kann auch eine Ausrichtung in Richtung proximal aufweisen. In diesem Fall ist die Membran zwar in ihrem Randbereich im Umfang des Implantats fixiert, der mittlere Bereich der Membran erstreckt sich aber in proximaler Richtung. Es ergibt sich somit insgesamt eine Kegel- oder Pyramidenform, wobei die Basis des Kegels/der Pyramide orthogonal zur Längsachse orientiert und die Membran im Randbereich mit dem Implantat verbunden ist, während der Scheitel des Kegels/der Pyramide weiter proximal liegt. Der Blutstrom wird, wenn er auf die Membran trifft, auf diese Weise geteilt und seitwärts abgelenkt, so dass der Blutstrom in das Aneurysma weitgehend zum Erliegen kommt.

Auch im Falle einer Kegel-/Pyramidenform der als Trennelement vorgesehenen Membran kann diese eine oder mehrere Ausnehmungen aufweisen, damit weiterhin nach Platzierung des Implantats durch die Ausnehmung hindurch Okklusionsmittel in das Aneurysma eingebracht werden können.

Um die Kegel-/Pyramidenform der Membran dauerhaft zu erhalten, sollte die Membran auf einer Gerüststruktur aus Fäden oder Drähten fixiert sein, wobei es sich prinzipiell auch um Stege handeln kann, die aus der das Implantat ausbildenden Struktur z. B. mit Hilfe eines Lasers herausgeschnitten sind. Dabei sollte darauf geachtet werden, dass die Fäden/Drähte eine hinreichende Steifigkeit aufweisen, um eine Umorientierung oder Umstülpung der Membran aufgrund des Blutdrucks zu verhindern. Ggf. müssen hierfür zusätzliche Fäden oder Drähte eingebracht werden.

Eine Möglichkeit besteht darin, ein Fadenkreuz aus zwei relativ langen Einzelfäden zu erzeugen, an dem die Membran fixiert ist, wobei aufgrund der Länge der Einzelfäden die Membran zunächst nicht gespannt ist. Ein oder mehrere Fäden können darüber hinaus an einer weiter proximal liegenden Schlaufe des Implantats fixiert sein, so dass das Fadenkreuz und damit die Membran in Richtung proximal gespannt werden, sobald das Implantat gestreckt wird. Selbstverständlich muss es sich nicht um lediglich zwei Fäden handeln, die ein Fadenkreuz ausbilden, ebenso denkbar sind nahezu beliebige andere Fadengeflechte, die eine Art Gerüststruktur ausbilden, um der Membran eine Struktur aufzuprägen.

Allgemein ist für die Erfindung wesentlich, dass der distale Abschnitt, ggf. mit dem Trennelement, seine Funktion erfüllt, nämlich in das Aneurysma eingeführte Okklusionsmittel, etwa Okklusionsspiralen, zuverlässig zurückzuhalten oder den Blutstrom so abzulenken, dass weitere Okklusionsmittel unnötig sind. Das Trennelement weist zumindest auch eine Komponente orthogonal zur Längsachse des Implantats auf.

Werden die Trennelemente durch das Einziehen von Fasern, Fäden oder dünnen Drähten ausgebildet, ist die Anordnung von Ösen im distalen Abschnitt zweckmäßig, in die die Fäden kreuz- oder sternförmig eingeknotet werden. Die Ösen selbst können aus einem Fasermaterial gefertigt sein. Die Fäden/Fasern bestehen z. B. aus einem geeigneten Polymer wie einem Polyamid (Nylon) oder es handelt sich um metallische Fasern.

Als Trennelemente können aber auch aus einem Rohrmaterial geschnittene Bögen oder (Draht)schlaufen genutzt werden, die in den Implantatkörper hinein umgebogen sind. Mindestens ein Bogen/eine Schlaufe ist hierfür erforderlich. Bei zwei bis vier Bögen/Schlaufen bilden diese ein stabiles Trennelement, das in ein Aneurysma eingebrachte Okklusionsmittel zuverlässig zurückhält.

Beim Kontrahieren des Implantats werden die Schlaufen typischerweise nach proximal gestreckt und legen sich an die weiteren Filamente des Implantats an, so dass sich das Implantat problemlos durch einen Katheter bewegen lässt. Zwischen den Schlaufen können schlitzförmige Öffnungen belassen werden, durch die Okklusionsmittel in das Aneurysma einführbar sind. Alternativ ist es aber auch möglich, die Schlaufen und/oder die Zwischenbereiche zwischen den Schlaufen mit einer Membran zu versehen, um ein möglichst dichtes Trennelement zu erzeugen. Auch die Verwendung von Membranen, die noch ein oder mehrere Öffnungen aufweisen, ist grundsätzlich möglich.

Zu den verschiedenen Möglichkeiten der Ausgestaltung der Trennelemente oder zum Versehen des distalen Abschnitts mit Membranen sei auch auf die WO 2014/029835 A1 verwiesen, deren Inhalt auch Gegenstand der Offenbarung der vorliegenden Erfindung sein soll.

Der distale Abschnitt des erfindungsgemäßen Implantats ist insbesondere atraumatisch, weich und elastisch ausgebildet. Die Wandungen von Aneurysmen sind empfindlich und können bei Belastung reißen, was unter allen Umständen verhindert werden muss. Entsprechend sollte insbesondere der distale Abschnitt des erfindungsgemäßen Implantats atraumatisch gestaltet sein. Dies wird beispielsweise mit der Anordnung von Schlaufen oder Bögen erreicht, die sich dort, wo sie Kontakt mit der Aneurysmawandung bekommen, sanft an diese anschmiegen. Solche Schlaufen oder Bögen können, wie andere Bereiche des Implantats auch, durch Laserschneiden aus einem Rohr generiert, mittels angefügter Drähte oder aus einem einheitlichen Drahtgeflecht erzeugt werden.

Im distalen Abschnitt sollten alle Drahtenden atraumatisch ausgebildet werden, um eine Perforation der Aneurysmawand zu verhindern.

Die erfindungsgemäßen Implantate können im Fixierabschnitt die Form eines aus einer Maschenstruktur gebildeten seitlich geschlossenen Rohrs haben, jedoch auch seitlich partiell oder durchgehend geschlitzt sein. Die Schlitzung kann achsparallel verlaufen oder schräg/helixförmig. In solch einem Fall ist in den geschlitzten Bereichen die Maschenstruktur entsprechend der Gefäßform gerollt, etwa in der Form eines gerollten Segments eines Maschendrahtzauns. Bei der Implantation eines solchen geschlitzten Implantats erlaubt dies eine gute Anpassung an das Gefäßlumen, insbesondere des zuführenden Gefäßes, wobei eine geringfügige Unter- oder Überdeckung der seitlichen Ränder der Maschenstruktur in der Regel unproblematisch ist. Überraschend hat sich gezeigt, dass die Schlitzung keinen negativen Einfluss auf die Radialkraft ausüben muss, auf der anderen Seite jedoch ein solches Implantat beim Vorschieben durch den Katheter weniger Widerstand erzeugt.

Es ist möglich, zumindest einige Maschen des Implantats im Fixierabschnitt mit Unterbrechungen zu versehen, d. h. die Maschen sind zum Teil nicht vollständig geschlossen. Ein solches Open-Cell-Design weist eine höhere Flexibilität auf, was bei stark gewundenen Blutgefäßen von Vorteil sein kann. Darüber hinaus bewirkt das Fehlen von Stegen/Streben einen verbesserten Blutfluss im Bereich der Gefäßverzweigung. Allerdings wird der Vorteil der erhöhten Flexibilität damit erkauft, dass sich ein Implantat mit Open-Cell-Design schlechter oder gar nicht in den Mikrokatheter zurückziehen lässt, wenn sich dies bei der Einführung als notwendig herausstellen sollte. Aus diesem Grund kann bei einer solchen Ausführungsform die proximale Anbindung an eine Einführhilfe entfallen. Ein alternatives Einfuhrsystem kann beispielsweise so aussehen, dass das im Mikrokatheter radial komprimierte Implantat auf einem Draht zwischen zwei Nocken lagert und sich bei Entfernung des Mikrokatheters selbständig entfaltet und dadurch vom Einfuhrsystem löst.

Die erfindungsgemäßen Implantate weisen in der Regel röntgendichte Markerelemente auf, die die Visualisierung und Platzierung am Implantationsort erleichtern. Insbesondere kann es sich bei der im Übergangsabschnitt vorgesehenen Hülse um ein solches Markerelement handeln. Darüber hinaus können Markerelemente beispielsweise im Bereich des distalen Endes des distalen Abschnitts angeordnet sein, wobei sie bei zusammengeführten Drähten die Verbindungspunkte atraumatisch umformen können. Solche Markerelemente können auch in Form von Drahtwicklungen, als Manschetten und als geschlitzte Rohrabschnitte vorliegen, die am Implantat festgelegt werden. Beispielsweise können als Markerelemente die die Schlaufen ausbildenden Filamente umgebende Markercoils vorgesehen sein, wobei in der Regel nicht die gesamten Schlaufen, sondern beispielsweise nur eine Hälfte von Markercoils umgeben ist. Für die Markerelemente kommen als Materialien insbesondere Platin und Platinlegierungen in Frage, beispielsweise eine Legierung aus Platin und Iridium, wie sie vielfach im Stand der Technik für Markerzwecke und als Material für Okklusionscoils eingesetzt wird. Idealerweise ist der distale Abschnitt und sind insbesondere die Schlaufen/Streben/Bögen im distalen Abschnitt vollständig oder teilweise röntgendicht, d. h. röntgensichtbar ausgebildet.

Möglich ist es auch, röntgensichtbare Substanzen in die Membranen einzubringen. Hierbei kann es sich um röntgensichtbare Partikel handeln, wie sie üblicherweise in der Röntgentechnik als Kontrastmittel eingesetzt werden. Solche röntgendichten Substanzen sind beispielsweise Schwermetallsalze wie Bariumsulfat oder lodverbindungen. Die Röntgensichtbarkeit der Membran ist bei der Einbringung und Lokalisierung des Implantats hilfreich und kann zusätzlich oder anstelle von Markerelementen Verwendung finden. Eine weitere Alternative besteht in einer teilweisen Goldbeschichtung von Bereichen des Implantats, beispielsweise der Schlaufen oder bestimmter Bereiche der Schlaufen.

Ggf. kann ein Teil des Implantats von Stegen mit dünnerem Querschnitt gebildet werden, um die Flexibilität des Implantats zu erhöhen. Der Bereich liegt vorzugsweise im Fixierabschnitt und soll einem nicht regelmäßigen Verlauf des Blutgefäßes in der Fixierzone Rechnung tragen.

Die Implantate müssen keine rohrförmige Struktur haben, sondern können auch als gerollte "Matten" vorliegen, die sich gegen die Gefäßwand aufspannen. Auch eine partielle Schlitzung ist möglich.

Die Erfindung betrifft schließlich ein Implantat nach der vorstehenden Beschreibung, das an einen Führungsdraht angekoppelt ist. Diese Ankopplung kann beispielsweise durch Verbindungselemente erfolgen, die sich unter Einwirkung von elektrischem Strom elektrolytisch auflösen. Solche Verbindungselemente und Materialien sind insbesondere für die Ablösung von Okklusionsspiralen und Stents vielfach beschrieben. Um die elektrolytische Ablösung zu beschleunigen und den Strom auf das Verbindungselement zu konzentrieren, ist es sinnvoll, wenn dieses gegenüber dem eigentlichen Implantat elektrisch isoliert ist. Alternativ kann auch das Implantat selbst zwar leitend mit dem Verbindungselement verbunden, jedoch insgesamt elektrisch isoliert sein, insbesondere durch einen elektrisch isolierenden Überzug. Hierzu bietet sich ein Kunststoffüberzug an, beispielsweise durch Verwendung von Parylen C.

Auch eine mechanische Ablösung durch Kupplungselemente ist ohne Weiteres möglich, wobei die Kupplungselemente mit entsprechend angepassten Kupplungsteilen des Führungsdrahts zusammenwirken. Unter dem äußeren Zwang eines Katheters oder einer Hülle bleibt diese Verbindung intakt; nach Herausschieben des Implantats und der Kupplungsstelle aus dem Katheter oder der Umhüllung löst sich aber die Verbindung und setzt das Implantat mit den zum Implantat gehörigen Kupplungselementen frei.

Eine weitere Variante ist die thermische Ablösbarkeit des Implantats.

Die Offenbarung betrifft auch ein Verfahren zur Einbringung des erfindungsgemäßen Implantats in das Blutgefäßsystem. Dies kann mit Hilfe eines üblichen Mikrokatheters erfolgen; diese Technik ist allgemein erprobt und wird vielfach angewandt. Sofern die Trennelemente nicht bereits selbst für eine ausreichende Abdichtung des Aneurysmahalses sorgen, werden nach Platzierung des Implantats Okklusionsmittel in das Aneurysma eingebracht. Hierzu wird das distale Ende eines Mikrokatheters in das Aneurysma geführt und die Okklusionsmittel, insbesondere Coils werden freigesetzt. Anschließend wird der Mikrokatheter zurückgezogen, die Okklusionsmittel werden durch das Implantat daran gehindert, aus dem Aneurysma zu entweichen. Neben herkömmlichen Okklusionsmitteln wie Coils können auch in anderer Weise geformte Körper zum Verschließen von Aneurysmen eingebracht werden, beispielsweise geflochtene oder auf andere Weise geformte Kugelkörper.

Die Erfindung wird durch die beiliegenden Abbildungen beispielhaft näher erläutert. Es zeigen:
- Figur 1: Ein Bifurkationsaneurysma in einer Prinzipskizze;
- Figur 2a: ein Implantat in der Seitenansicht;
- Figur 2b: ein weiteres Implantat in der Seitenansicht;
- Figur 2c: ein weiteres Implantat in der Seitenansicht;
- Figur 2d: ein nicht erfindungsgemäßes Implantat in der Seitenansicht;
- Figur 3: ein Implantat in einer Ansicht von proximal;
- Figur 4: ein weiteres Implantat in einer Ansicht von distal;
- Figur 5: eine Prinzipdarstellung eines nicht erfindungsgemäßen Implantats hinsichtlich der Winkel, die die Schlaufen im distalen Abschnitt ausbilden;
- Figur 6a,b: eine Variante eines erfindungsgemäßen Implantats mit einer Öse zur Fixierung der Hülse;
- Figur 7a,b: ein Implantat mit Membranbespannung der Schlaufen in der Seitenansicht sowie einer Ansicht von distal; und
- Figur 8: eine weitere Variante einer Ausführungsform in der Seitenansicht.

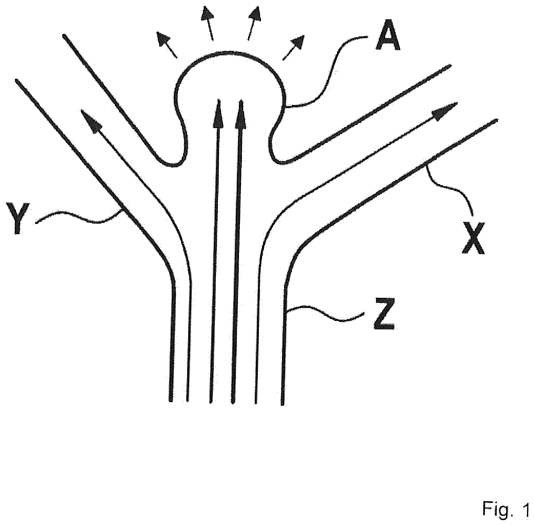

Figur 1 zeigt ein Bifurkationsaneurysma mit einem zuführenden Gefäß Z, zwei abführenden Gefäßen X und Y sowie dem in der Gabelung angeordneten Aneurysma A. Die langen Pfeile stellen den Blutstrom dar, der auf der Prallseite in das Aneurysma A einfließt und dort einen Druck nach außen ausübt, unter dem das Aneurysma sich erweitert (kleine Pfeile).

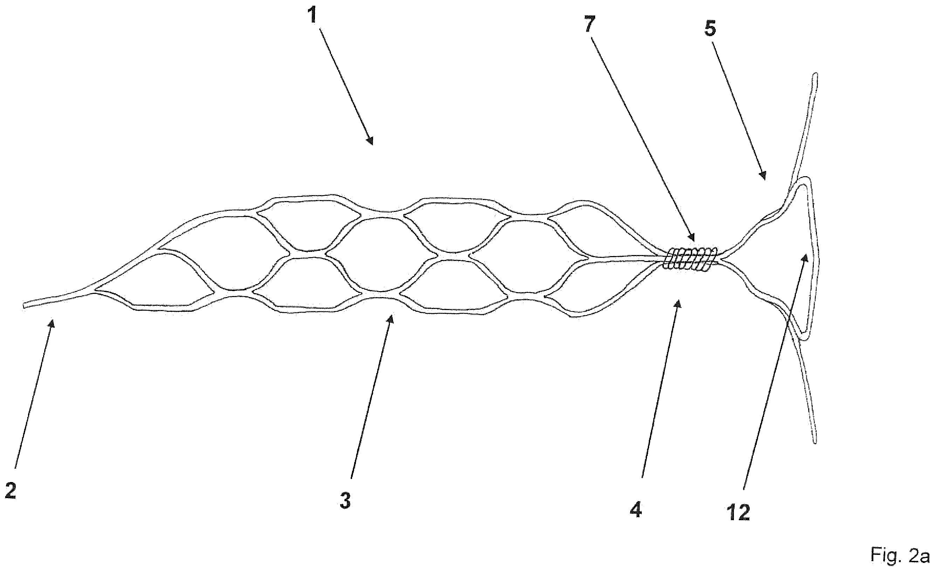

Figur 2a zeigt ein Implantat 1 im expandierten Zustand in der Seitenansicht. Das Implantat 1 weist einen Fixierabschnitt 3 und einen distalen Abschnitt 5 auf, wobei der distale Abschnitt 5 radial gegenüber dem Fixierabschnitt 3 erweitert ist. Dabei bildet er vier Schlaufen 12 aus, die sich im Inneren des Aneurysmas an dessen Wandung anlegen, wobei zusätzlich hier nicht dargestellte Trennelemente vorgesehen sind, die den Aneurysmahals soweit verschließen, dass in das Aneurysma eingebrachte Okklusionsmittel nicht wieder austreten können.

Zwischen dem Fixierabschnitt 3 und dem distalen Abschnitt 5 ist ein Übergangsabschnitt 4 vorgesehen, der einen kleinen Querschnitt aufweist. Von proximal (in der Zeichnung links) nach distal (in der Zeichnung rechts) gesehen werden die das Implantat 1 ausbildenden Filamente vom Fixierabschnitt 3 ausgehend im Übergangsabschnitt 4 eng zusammengeführt und erweitern sich anschließend wieder zum distalen Abschnitt 5. Im Übergangsabschnitt 4 ist eine Hülse 7 angeordnet, durch die die Filamente verlaufen und die die Filamente zusammenhält. Die im Übergangsabschnitt 4 verlaufenden Filamente sind in gewissem Maße zueinander beweglich, können sich jedoch nicht weiter radial ausdehnen. Auf diese Weise wird eine hohe Flexibilität des Implantats 1 um den Übergangsabschnitt 4 herbeigeführt, sodass der distale Abschnitt 5 sich auch an unregelmäßige Formen eines Aneurysmas A anpassen kann.

Im konkreten Beispiel ist die Hülse 7 als Drahtwendel ausgebildet. Diese ist aus einem röntgensichtbaren (röntgenstrahlungsundurchlässigen) Material gefertigt und ermöglicht dem behandelnden Arzt auf diese Weise die Visualisierung des Implantats 1 während der Einführung. Am proximalen Ende des Fixierabschnitts 3 läuft das Implantat 1 in einem Kupplungselement 2 in Form eines Kupplungsdrahtes aus, über welches das Implantat 1 mit einer Einführhilfe, insbesondere einem Einführdraht verbunden ist.

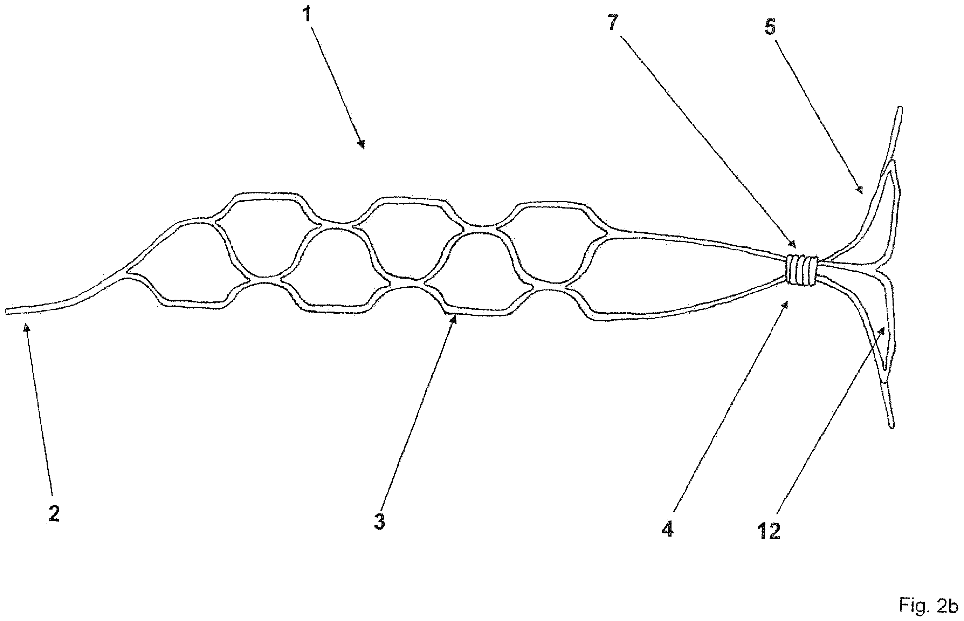

In Figur 2b ist ein abgewandeltes Implantat 1 dargestellt, wobei die Darstellung im Wesentlichen der aus Figur 2a entspricht. Allerdings ist der Fixierabschnitt 3 nicht über die gesamte Länge wabenförmig ausgebildet, der distale Bereich des Fixierabschnitts 3 besteht aus in Richtung des Übergangsabschnitts 4 weitgehend gerade verlaufenden Filamenten. Im Fixierabschnitt 3 beträgt die Zahl der von den Filamenten ausgebildeten Waben in einer orthogonal zur Längsachse des Implantats 1 verlaufenden Schnittebene jeweils drei, d.h. in jeder Schnittebene sind über den Umfang des Implantats 1 drei Waben nebeneinander angeordnet. Auf der anderen Seite ist die Zahl der im distalen Abschnitt 5 angeordneten Schlaufen 12 höher; insgesamt werden in diesem Ausführungsbeispiel sechs Schlaufen 12 ausgebildet.

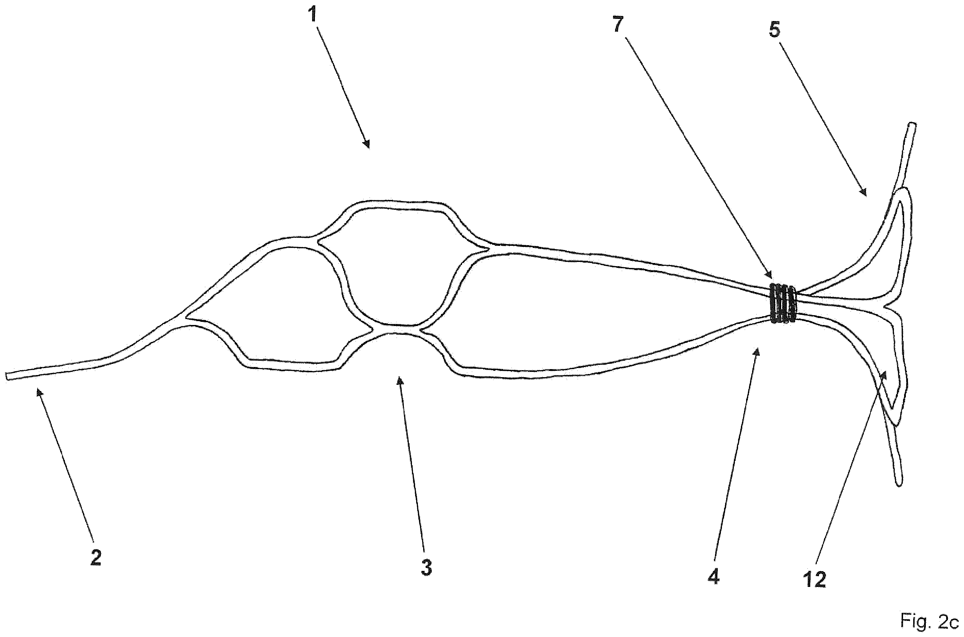

In Figur 2c ist eine weitere Ausführungsform des Implantats 1 dargestellt, die wiederum im Wesentlichen der Darstellung aus den Figuren 2a und 2b entspricht. Allerdings ist hier der Fixierabschnitt 3 sehr kurz gehalten und umfasst nur einen Kranz von Waben. Dies kann insbesondere dann von Vorteil sein, wenn der distale Abschnitt 5 mit den Schlaufen 12 nicht im, sondern vor dem Aneurysma platziert werden soll. Wie im Ausführungsbeispiel nach Figur 2b beträgt die Zahl der Waben im Fixierabschnitt 3 entlang einer Umfangslinie drei und die Zahl der Schlaufen 12 insgesamt sechs.

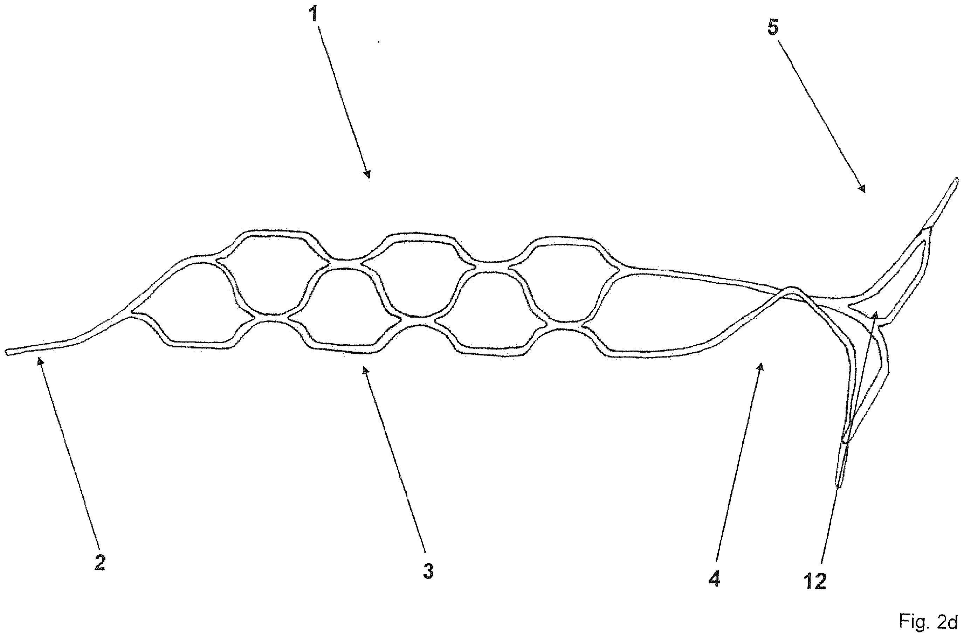

In Figur 2d schließlich ist eine nicht erfindungsgemäße Variante dargestellt, die sich dadurch von den Ausführungsformen der Figuren 2a bis 2c unterscheidet, dass der Übergangsabschnitt 4 keine Hülse 7 aufweist. Wie in der Zeichnung angedeutet, kann dies dazu führen, dass bei abknickendem distalen Abschnitt 5, wie es bei unregelmäßig geformten Aneurysmen häufig der Fall ist, die Filamente im Übergangsabschnitt 4 nach außen gespreizt werden und nicht mehr unmittelbar nebeneinander verlaufen. Dieser Effekt kann sich noch stärker bemerkbar machen als in der Zeichnung dargestellt und dann ggf. zu einem unvorteilhaften Sitz des Implantats 1 im Blutgefäß bis hin zu einer Verletzung der Gefäßwand durch sich bildende scharfe Knickpunkte führen. Dieser Effekt wird erfindungsgemäß durch das Vorsehen einer Hülse 7 vermieden.

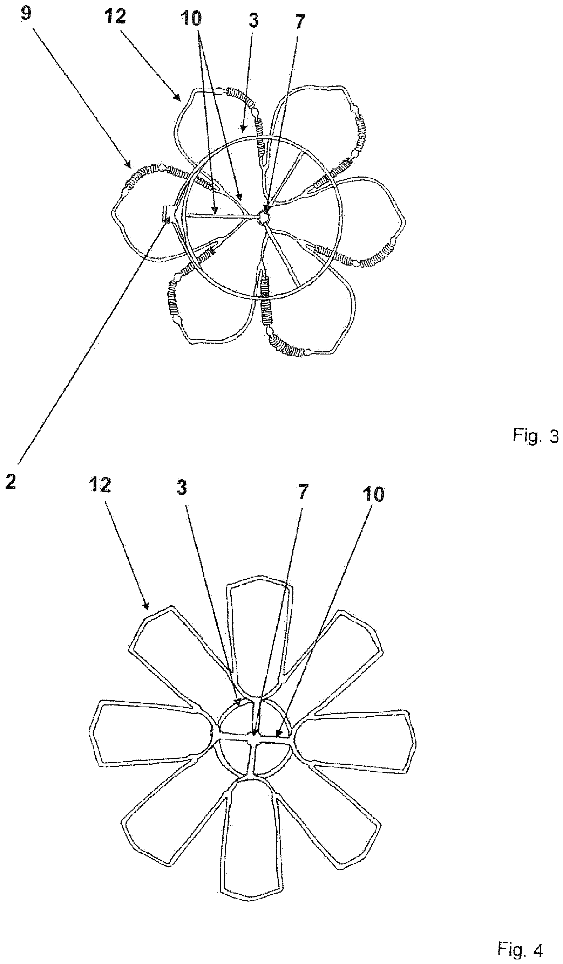

In Figur 3 ist ein Implantat 1 in einer Ansicht von proximal im expandierten Zustand dargestellt. Man erkennt, dass der distale Abschnitt 5 mehrere Schlaufen 12 ausbildet, die nach radial außen stehen und für eine Verankerung im Aneurysma sorgen. Der distale Abschnitt 5 hat somit eine Blütenform. Die Schlaufen 12 sind jeweils einseitig mit einer eng gewickelten Markercoil 9 aus einem röntgensichtbaren Material versehen, die die Sichtbarkeit des Implantats 1 für den behandelnden Arzt verbessert. Der Fixierabschnitt 3 ist hier, betrachtet von proximal, angedeutet als Kreis zu erkennen, wobei im Randbereich des Fixierabschnitts exzentrisch ein Kupplungselement 2 zur Verbindung mit der Einführhilfe vorgesehen ist. Im Übergangsabschnitt laufen die Filamente 10 sowohl vom distalen Abschnitt 5 als auch vom Fixierabschnitt 3 aus im Zentrum zusammen durch die Hülse 7.

In Figur 4 ist ein weiteres Implantat 1 in einer Ansicht von distal im expandierten Zustand dargestellt, das sich u. a. dadurch vom Implantat 1 aus Figur 3 unterscheidet, dass ingesamt acht Schlaufen 12 vorgesehen sind. Im Hintergrund erkennt man den im Querschnitt kreisförmigen Fixierabschnitt 3, von dem aus die Filamente zentral im Übergangsabschnitt durch die Hülse 7 zusammenlaufen. Von dort aus erstrecken sich die Filamente 10 und Schlaufen 12 radial nach außen, um den distalen Abschnitt 5 auszubilden und den Aneurysmahals soweit zu verschließen, dass in das Aneurysma eingebrachte Okklusionsmittel am Austritt gehindert werden bzw. das Aneurysma so weit wie möglich vom Blutfluss abgeschnitten wird.

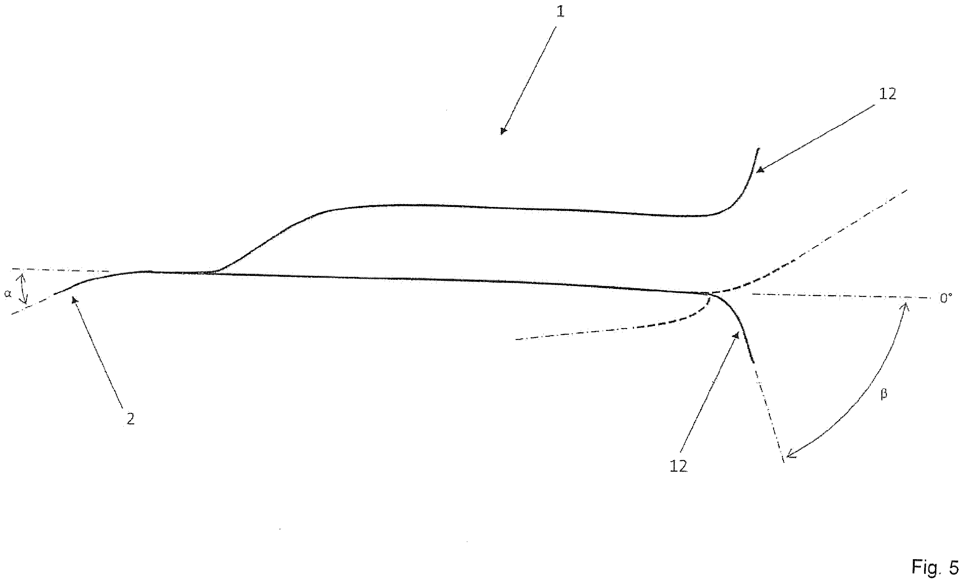

Figur 5 zeigt schematisch ein Implantat 1, das hinsichtlich der Abwesenheit eines sehr schmalen, von einer Hülse umgebenen Übergangsabschnitts zwar nicht als erfindungsgemäß anzusehen ist, jedoch die Ausbildung unterschiedlicher Winkel β der hier nur schematisch wiedergegebenen Schlaufen 12 zur Längsachse des Implantats 1 verdeutlicht. Die Längsachse ist als gestrichelte Linie dargestellt. Der Winkel β kann sehr groß sein (> 90°, gestrichelte Darstellung), was insbesondere bei stark ausgewölbten Aneurysmen A von Vorteil ist, wobei die Auswölbung zumindest partiell in Richtung proximal verläuft. In Extremfällen kann der Winkel β nahezu 180° betragen. Auf diese Weise wird eine gute Anschmiegung des distalen Abschnitts 5 an die Aneurysmawand erreicht.

In anderen Fällen (ebenfalls gestrichelt dargestellt) kann es von Vorteil sein, wenn der Winkel β negativ ist, weil ein Teil der Aneurysmawand nach innen gekrümmt ist. Wichtig ist, dass die Winkel für die einzelnen Schlaufen 12 oder auch Streben sich unterscheiden können, was bei unregelmäßig geformten Aneurysmen A von großem Vorteil ist.

Daneben ist in Figur 5 ebenfalls zu erkennen, dass das proximale Ende 2 des Implantats 1, an dem das Implantat 1 in Kupplungsdrähten ausläuft, über die das Implantat 1 mit einer Einführhilfe verbunden ist, einen Winkel α zur Längsachse des Implantats 1 ausbildet. Dieser Winkel liegt u. U. nur im vollständig expandierten Zustand ohne äußeren Zwang vor. Auf diese Weise wird eine verbesserte Expansion des Implantats 1 und das Anlegen an die Blutgefäßwand gefördert. Ein unerwünschtes Hineinragen in das Blutgefäß Z wird vermieden.

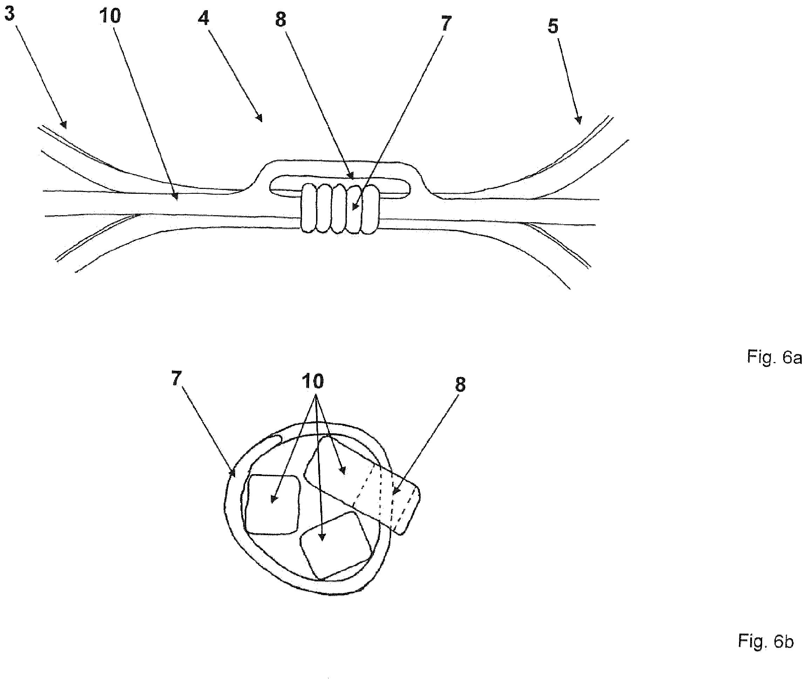

Figur 6a zeigt den Übergangsabschnitt 4 eines erfindungsgemäßen Implantats 1 in der Seitenansicht, bei dem eines der Filamente 10 im Bereich der Hülse 7 eine Öse 8 aufweist. Die Hülse 7 verläuft durch die Öse 8, sodass die Längsverschiebbarkeit der Hülse 7 eingeschränkt ist. Maximal kann sich die Hülse 7 bis zum proximalen bzw. distalen Anschlag der Öse 8 verschieben; eine weitergehende Verlagerung, die unter Umständen die korrekte Entfaltung des Implantats 1 behindern könnte, ist ausgeschlossen.

In Figur 6b ist ein Querschnitt der Hülse 7 im Übergangsabschnitt 4 dargestellt, wobei drei Filamente 10 durch die Hülse 7 verlaufen, von denen ein Filament 10 im Bereich der Hülse 7 breiter ausgebildet ist und eine Öse 8 aufweist, durch die die Hülse 7 verlaufen kann.

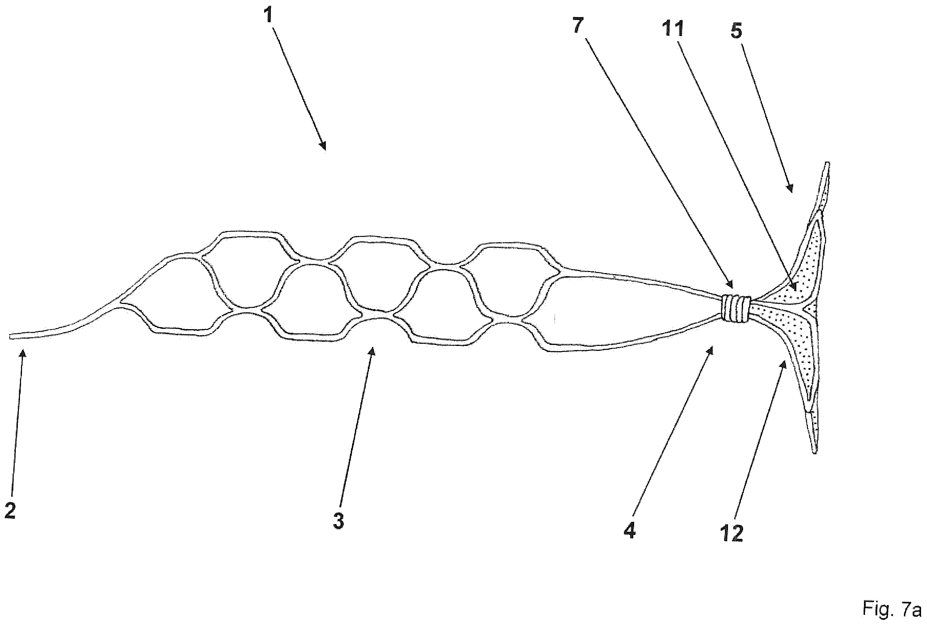

In Figur 7a ist eine Ausführungsform des Implantats 1 in der Seitenansicht dargestellt, das im Wesentlichen der Darstellung aus Figur 2b entspricht. Hiervon unterscheidet sich die Ausführungsform dadurch, dass zwischen den die einzelnen Schlaufen 12 ausbildenden Filamenten eine Membran 11 aufgespannt ist. Die Membranen 11 sind gepunktet dargestellt. Zum Teil sind auch Bereiche zwischen den Schlaufen 12 mit einer Membran 11 versehen. Es ist sowohl möglich, zwischen den Schlaufen 12 einzelne Membranen 11 als auch eine einzelne, sich über sämtliche Schlaufen 12 erstreckende Membran 11 vorzusehen. Die Membran 11 verläuft zentral trichterförmig in Richtung proximal und sorgt für die weitere Abkopplung des Aneurysmas vom Blutfluss.

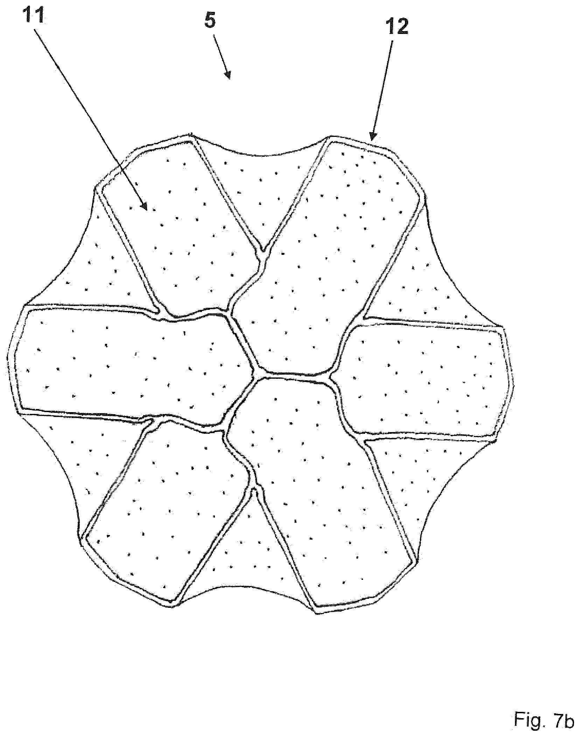

In Figur 7b ist dieselbe Ausführungsform in einer Ansicht von distal dargestellt, in der man erkennt, dass die einzelnen Schlaufen 12, aber auch Bereiche zwischen den Schlaufen 12 mit einer Membran 11 versehen sind.

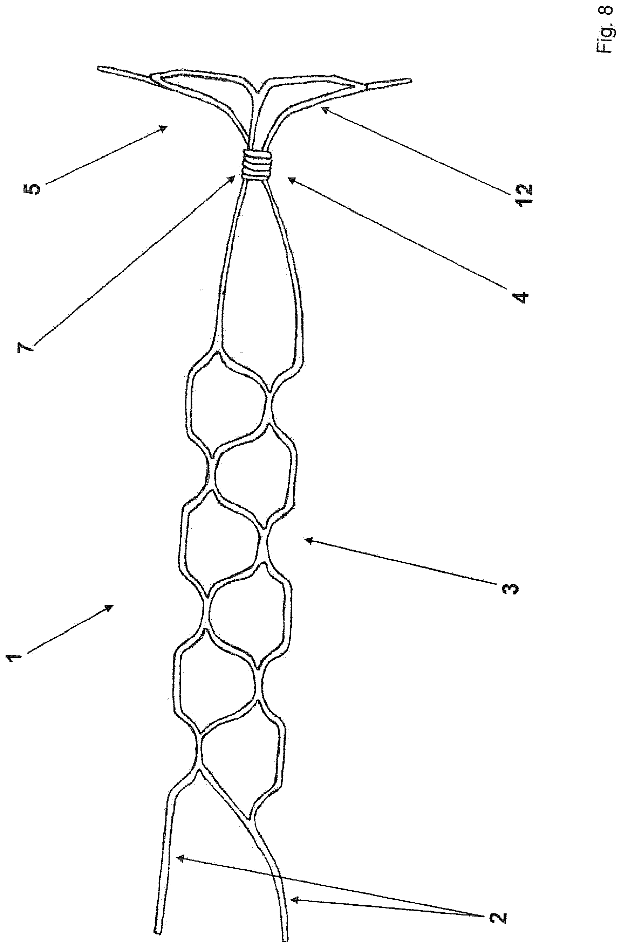

In Figur 8 ist eine weitere Variante der Ausführungsform aus Figur 2b dargestellt, die sich dadurch auszeichnet, dass zwei Kupplungselemente 2 vorgesehen sind, die sich beide jeweils im Randbereich des Fixierabschnitts 3 an dessen proximalen Ende befinden. Über die Kupplungselemente 2 ist das Implantat 1 an eine hier nicht dargestellte Einführhilfe gekoppelt. Das Vorsehen von mehr als einem Kupplungselement 2 verbessert die Rückziehbarkeit des Implantats, insbesondere dann, wenn der Fixierabschnitt 3 sehr kurz ist.

## Patentansprüche

1. Implantat zum Einsatz bei der Okkludierung von Aneurysmen (A) in Blutgefäßen (Z) im Bereich von Gefäßverzweigungen (X, Y), insbesondere Bifurkationsaneurysmen, wobei das Implantat (1) in einem expandierten Zustand, in dem es im Blutgefäß (Z) implantiert wird, und in einem kontrahierten Zustand vorliegt, in dem es durch das Blutgefäß (Z) bewegbar ist, wobei das Implantat (1) einen proximalen Fixierabschnitt (3), mit dem das Implantat (1) an einer Gefäßwand des Blutgefäßes (Z) fixierbar ist, einen distalen Abschnitt (5), in dem das Implantat (1) gegenüber dem Fixierabschnitt (3) radial erweitert ist und der zur Platzierung im oder vor dem Aneurysma (A) bestimmt ist, und einen Übergangsabschnitt (4) zwischen dem Fixierabschnitt (3) und dem distalen Abschnitt (5) aufweist, wobei das Implantat (1) aus miteinander verbundenen oder sich kreuzenden Filamenten (10) aufgebaut ist und im Übergangsabschnitt (4) ein oder mehrere vom Fixierabschnitt (3) oder distalen Abschnitt (5) ausgehende Filamente (10) zentral zusammenkommen, wobei die Filamente (10) im Übergangsabschnitt (4) zumindest bereichsweise durch eine Hülse (7) verlaufen, **dadurch gekennzeichnet, dass** ein oder mehrere Filamente (10) im Übergangsabschnitt (4) eine Öse (8) aufweisen, wobei die Hülse (7) zumindest abschnittsweise durch die Öse (8) verläuft und die Öse (8) die Hülse (7) in ihrer Beweglichkeit in Längsrichtung des Implantats (1) einschränkt.

2. Implantat nach Anspruch 1, **dadurch gekennzeichnet, dass** die Hülse (7) aus einer Drahtwendel mit einem inneren Hohlraum gebildet ist.

3. Implantat nach Anspruch 1 oder 2, **dadurch gekennzeichnet, dass** die Hülse (7) zumindest teilweise aus einem röntgensichtbaren Material gefertigt ist.

4. Implantat nach einem der Ansprüche 1 bis 3, **dadurch gekennzeichnet, dass** im Übergangsabschnitt (4) mehrere Filamente (10) durch die Hülse (7) verlaufen.

5. Implantat nach Anspruch 4, **dadurch gekennzeichnet, dass** die Filamente (10) im Übergangsabschnitt (4) parallel zueinander verlaufen.

6. Implantat nach einem der Ansprüche 1 bis 5, **dadurch gekennzeichnet, dass** proximal und/oder distal der Hülse (7) Stopper angeordnet sind, die ein Verschieben oder Verrutschen der Hülse (7) über die Stopper hinaus verhindern.

7. Implantat nach einem der Ansprüche 1 bis 6, **dadurch gekennzeichnet, dass** der distale Abschnitt (5) eine Mehrzahl von Streben, Schlaufen (12) oder Bögen aufweist, die zumindest teilweise nach radial außen weisen.

8. Implantat nach Anspruch 7, **dadurch gekennzeichnet, dass** die Streben, Schlaufen (12) oder Bögen zur Längsachse des Implantats (1) einen Winkel β zwischen -45° und +175°, vorzugsweise zwischen +45° und +90°, ausbilden, wobei ein positiver Winkel β für nach radial außen und ein negativer Winkel β für nach radial innen weisende Streben, Schlaufen (12) oder Bögen steht.

9. Implantat nach Anspruch 7 oder 8, **dadurch gekennzeichnet, dass** die Schlaufen (12) oder Bögen im Inneren mit einer Membran (11) versehen sind oder zwischen den Streben eine Membran aufgespannt ist.

10. Implantat nach einem der Ansprüche 1 bis 6, **dadurch gekennzeichnet, dass** der distale Abschnitt (5) kugelförmig, pilzförmig, ankerförmig oder ellipsoidförmig radial erweitert ist.

11. Implantat nach einem der Ansprüche 1 bis 10, **dadurch gekennzeichnet, dass** im distalen Abschnitt (5) zentral ein oder mehrere Trennelemente angeordnet sind, die im implantierten Zustand den Hals des Aneurysmas (A) zumindest teilweise verschließen.

12. Implantat nach Anspruch 11, **dadurch gekennzeichnet, dass** die Trennelemente aus Fasern, Fäden, Drähten oder Membranen (11) gebildet werden.

13. Implantat nach Anspruch 12, **dadurch gekennzeichnet, dass** die Trennelemente eine Membran (11) beinhalten und sich die Membran (11) in proximaler Richtung erstreckt und vorzugsweise eine Kegel- oder Pyramidenform ausbildet.

14. Implantat nach Anspruch 12 oder 13, **dadurch gekennzeichnet, dass** die Trennelemente eine Membran (11) beinhalten und die Membran (11) eine oder mehrere Öffnungen aufweist oder in der Membran (11) eine oder mehrere Öffnungen mittels Durchstechen erzeugbar sind.

## Claims

1. Implant to be used for the occlusion of aneurysms (A) in blood vessels (Z) in the region of vascular branches (X, Y), in particular bifurcation aneurysms, with the implant (1) being in an expanded state in which it is implanted in the blood vessel (Z) and in a contracted state in which it is movable through the blood vessel (Z), with the implant (1) having a proximal fixing section (3) by means of which the implant (1) can be secured to the wall of a blood vessel (Z), a distal section (5) where the implant (1) is radially widened relative to the fixing section (3) and which is intended for placement in or in front of the aneurysm (A), and having a transition section (4) located between the fixing section (3) and the distal section (5), wherein the implant (1) is composed of interconnected or intersecting filaments (10) and in the transition section (4) one or more filaments (10) starting from the fixing section (3) or distal section (5) come together centrally, the filaments (10) in the transition section (4) running at least in some areas through a sleeve (7),
**characterized in that**
one or more filaments (10) have an eyelet (8) in the transition section (4), the sleeve (7) extending at least in sections through the eyelet (8) and the eyelet (8) restricting the sleeve (7) in its mobility in the longitudinal direction of the implant (1).

2. Implant according to claim 1, **characterized in that** the sleeve (7) is formed of a wire coil with an inner cavity.

3. Implant according to claim 1 or 2, **characterized in that** the sleeve (7) is made at least partially of a radiopaque material.

4. Implant according to any of claims 1 to 3, **characterized in that** in the transition section (4) several filaments (10) are extending through the sleeve (7).

5. Implant according to claim 4, **characterized in that** the filaments (10) in the transition section (4) run parallel to each other.

6. Implant according to any of claims 1 to 5, **characterized in that** stoppers are arranged proximally and/or distally of the sleeve (7) which prevent the sleeve (7) from sliding or slipping beyond the stoppers.

7. Implant according to any of claims 1 to 6, **characterized in that** the distal section (5) comprises a plurality of struts, loops (12) or arches that at least partially are facing radially outward.

8. Implant according to claim 7, **characterized in that** the struts, loops (12) or arches form an angle β ranging between -45° and +175°, preferably between +45° and +90° in relation to the longitudinal axis of the implant (1), wherein a positive angle β stands for struts, loops (12) or arches pointing radially outwards and a negative angle β for struts, loops (12) or arches pointing radially inwards.

9. Implant according to claim 7 or 8, **characterized in that** the loops (12) or arches are provided inside with a membrane (11) or that a membrane is spanned between the struts.

10. Implant according to any of claims 1 to 6, **characterized in that** the distal section (5) is radially widened so as to from a spherical, mushroom, anchor, or ellipsoidal shape.

11. Implant according to any of claims 1 to 10, **characterized in that** one or several separation elements are arranged centrally in the distal section (5), said separation element at least partially occluding the neck of the aneurysm (A) in the implanted state.

12. Implant according to claim 11, **characterized in that** the separation elements are formed from fibers, threads, wires or membranes (11).

13. Implant according to claim 12, **characterized in that** the separation elements comprise a membrane and the membrane (11) extends in the proximal direction and, preferably, has a conical or pyramid form.

14. Implant according to claim 12 or 13, **characterized in that** the separation elements comprise a membrane and the membrane (11) has one or more openings or one or more openings can be produced in the membrane (11) by a piercing method.

## Revendications

1. Implant destiné à être employé lors de l'occlusion d'anévrismes (A) dans des vaisseaux sanguins (A) dans la zone de ramifications vasculaires (X, Y), en particulier d'anévrismes de bifurcation, dans lequel l'implant (1) se trouve dans un état expansé, dans lequel il est implanté dans le vaisseau sanguin (Z), et dans un état contracté, dans lequel il peut être déplacé à travers le vaisseau sanguin (Z), dans lequel l'implant (1) présente une section de blocage proximale (3), avec laquelle l'implant (1) peut être bloqué au niveau d'une paroi de vaisseau du vaisseau sanguin (Z), une section distale (5), dans laquelle l'implant (1) est élargi radialement par rapport à la section de blocage (3) et qui se destine à être placée dans ou devant l'anévrisme (A), et une section de transition (4) entre la section de blocage (3) et la section distale (5), dans lequel l'implant (1) est élaboré à partir de filaments (10) reliés entre eux ou se croisant et un ou plusieurs filaments (10) partant de la section de blocage (3) ou de la section distale (5) se réunissent au centre dans la section de transition (4), dans lequel les filaments (10) s'étendent dans la section de transition (4) au moins par endroits à travers une douille (7), **caractérisé en ce qu'**un ou plusieurs filaments (10) présentent dans la zone de transition (4) un œillet (8), dans lequel la douille (7) s'étend au moins par endroits à travers l'œillet (8) et l'œillet (8) restreint la douille (7) dans sa possibilité de déplacement dans le sens longitudinal de l'implant (1).

2. Implant selon la revendication 1, **caractérisé en ce que** la douille (7) est formée à partir d'un fil enroulé avec un espace creux intérieur.

3. Implant selon la revendication 1 ou 2, **caractérisé en ce que** la douille (7) est fabriquée au moins en partie à partir d'un matériau visible aux rayons X.

4. Implant selon l'une quelconque des revendications 1 à 3, **caractérisé en ce que** plusieurs filaments (10) s'étendent à travers la douille (7) dans la section de transition (4).

5. Implant selon la revendication 4, **caractérisé en ce que** les filaments (10) s'étendent de manière parallèle les uns par rapport aux autres dans la section de transition (4).

6. Implant selon l'une quelconque des revendications 1 à 5, **caractérisé en ce que** sont disposés de manière proximale et/ou de manière distale par rapport à la douille (7) des arrêtoirs, qui empêchent un coulissement ou un glissement de la douille (7) au-delà des arrêtoirs.

7. Implant selon l'une quelconque des revendications 1 à 6, **caractérisé en ce que** la section distale (5) présente une multitude d'entretoises, de boucles (12) ou d'arcs, qui pointent au moins en partie vers l'extérieur radialement.

8. Implant selon la revendication 7, **caractérisé en ce que** les entretoises, les boucles (12) ou les arcs forment par rapport à l'axe longitudinal de l'implant (1) un angle β entre -45° et +175°, de préférence entre +45° et +90°, dans lequel un angle β positif représente des entretoises, des boucles (12) ou des arcs pointant vers l'extérieur radialement et un angle β négatif représente des entretoises, des boucles ou des arcs pointant vers l'intérieur radialement.

9. Implant selon la revendication 7 ou 8, **caractérisé en ce que** les boucles (12) ou les arcs sont pourvus à l'intérieur d'une membrane (11) ou une membrane est tendue entre les entretoises.

10. Implant selon l'une quelconque des revendications 1 à 6, **caractérisé en ce que** la section distale (5) est élargie radialement de manière à présenter une forme de sphère, une forme de champignon, une forme d'ancre ou une forme d'ellipsoïde.

11. Implant selon l'une quelconque des revendications 1 à 10, **caractérisé en ce que** sont disposés dans la section distale (5) au centre un ou plusieurs éléments de séparation, qui ferment au moins en partie dans l'état implanté le col de l'anévrisme (A).

12. Implant selon la revendication 11, **caractérisé en ce que** les éléments de séparation sont formés à partir de fibres, de fils, de fils métalliques ou de membranes (11).

13. Implant selon la revendication 12, **caractérisé en ce que** les éléments de séparation renferment une membrane (11) et la membrane s'étend dans une direction proximale et réalise de préférence une forme de cône ou de pyramide.

14. Implant selon la revendication 12 ou 13, **caractérisé en ce que** les éléments de séparation renferment une membrane (11) et la membrane (11) présente une ou plusieurs ouvertures et une ou plusieurs ouvertures peuvent être produites dans la membrane (11) au moyen d'un transpercement.
